# EUROPEAN PATENT APPLICATION

(11) **EP 0 601 979 A2**
(43) Date of publication of application: **15.06.1994**
(21) Application number: 93830085.2
(22) Date of filing: 03.03.1993
(51) Int. Cl.: A61K 39/385, A61K 9/127, A61K 39/21, A61K 39/015, A61K 39/002

(54) **Exogenous antigens bound to hepatitis B virus envelope antigen**

(30) Priority: 10.12.1992 IT RM920877
(71) Applicant: FONDAZIONE ANDREA CESALPINO, I-00161 Roma (IT)
(72) Inventor: Balsano, Francesco, I-00198 Roma RM (IT); Barnaba, Vincenzo, I-00199 Roma RM (IT)
(74) Representative: Di Cerbo, Mario

(57) **Abstract**

The process for the preparation of immunogens according to the invention is characterized by synthetically bonding at least one exogenous antigen to the exogenous form of hepatitis B virus envelope antigen, such immunogens being capable of eliciting, against the synthetically bonded antigen, not only an immune response of T helper lymphocytes (class II-restricted) but also an effective response of cytotoxic T lymphocytes (class I-restricted). The synthetically bonded antigens can be selected for example from the group comprising proteins of AIDS virus, recombinant proteins of AIDS virus, synthetic peptides of AIDS virus, proteins of malaria plasmodium and proteins of toxoplasma. The invention extends also to immunogens per se, characterized by the fact of being obtainable with the above process.

## Description

The present invention refers to a process for the preparation of immunogens which comprises synthetically bonding at least one exogenous antigen to the exogenous form of hepatitis B virus envelope antigen (hereinafter also referred to as HBenvAg). Such immunogens are capable of eliciting, against the synthetically bonded antigen, not only an immune response of T helper lymphocytes (class II-restricted) but also an effective response of cytotoxic T lymphocytes (class I-restricted). It must be emphasized that these last cells are fundamental for the clearance of cells infected by pathogenic agents.

As it is known, the recognition of antigen by T lymphocytes can be differentiated from the recognition by B lymphocytes for two main reasons:
a) In contrast to B cells, T cells do not recognize the antigen in its native conformation, but only peptidic fragments derived from its processing by antigen presenting cells (APC);
b) such peptides are recognized only if associated with other membrane molecules of APC, constituted by the products of major histocompatibility complex (MHC) genes.

T lymphocytes with CD4+ phenotype recognize the antigen in association with class II MHC molecules. On the, contrary, T lymphocytes with CD8+ phenotype recognize the antigen in association with class I MHC molecules.

The experimental evidence suggests the existence of two separate pathways of processing and presentation of antigen. The separate pathways lead to the selective association of endogenous cellular antigens to class I molecules, and of exogenous antigens to class II molecules. Consequently, CD4+ class II-restricted T cells are usually induced by exogenous soluble antigens, while CD8+ class I-restricted T cells are generally induced by endogenously synthesized antigens, such as those expressed by virus infected cells, or by cellular self antigens.

Summarizing, exogenous antigens, which enter into the APC through a fluid phase mechanism or by receptor-mediated endocytosis, are processed by acid proteases at the endosomal level and seem to have a selective access to class II molecules, as a result of a vescicular fusion at the level of the trans- or post-Golgi region. On the contrary, peptides derived from proteins synthesized by the cells themselves would be processed in the cytosol (probably from enzymes of cis-medial-Golgi but not of trans-Golgi) and could preferentially interact with class I molecules at the level of endoplasmic reticulum or of the cis-Golgi region.

All that has a precise biological sense: for example, during a viral infection, cytotoxic class I-restricted T cells will destroy the virus infected cells, while helper class II-restricted T cells will induce an antibody response to neutralize exogenous viral particles or toxins.

The present invention is based on the discovery that HBenvAg, unexpectedly, can be presented to class I-restrictecd T cells and therefore can be used as a vector to cause other exogenous antigens to be processed and presented to class I-restricted T lymphocytes, when they are synthetically bonded to HBenvAg.

The present invention refers to a process for the preparation of immunogens characterized by synthetically bonding at least one exogenous antigen to the exogenous form of hepatitis B virus envelope antigen, such immunogens being capable of eliciting, against the synthetically bonded antigen, not only an immune response of T helper lymphocytes (class II-restricted) but also an effective response of cytotoxic T lymphocytes (class I-restricted).

According to specific variants of the process of the present invention, the above immunogens can be bonded to or incorporated into a lipoprotein membrane particle. The lipoprotein membranes can be liposomes.

According to other embodiments of the process of the present invention, the exogenous antigens synthetically bonded to the HBenvAg can be selected from the group comprising proteins of AIDS virus (HIV) recombinant proteins of AIDS virus, synthetic peptides of AIDS virus, proteins of malaria plasmodium and proteins of toxoplasma.

The present invention is not limited to the process for preparation of immunogens, but extends to the immunogens per se.

Subjects of the present invention are therefore also immunogens characterized by consisting of at least one exogenous antigen synthetically bonded to the exogenous form of hepatitis B virus envelope antigen. Such immunogens are capable of eliciting, against the synthetically bonded antigen, not only an immune response of T helper (class II-restricted), but also an effective response of cytotoxic T lymphocytes (class I-restricted).

The above immunogens can be bonded to or incorporated into lipoprotein membrane particles. The lipoprotein membranes can be liposomes.

According to other embodiments of the immunogens of the present invention, the antigens synthetically bonded to the exogenous form of hepatitis B virus envelope antigen can be selected for example from the group comprising proteins of AIDS virus (HIV), recombinant proteins of AIDS virus, synthetic peptides of AIDS virus, proteins of malaria plasmodium and proteins of toxoplasma.

So far, a general description of the invention forming the subject matter of the present patent has been given. To better understand its purposes, characteristics and advantages, a more detailed description will now be given with the help of the following example.

The example has only an explanatory function. Indeed the scope of the invention is substantially defined only by the following claims.

### EXAMPLE

### GENERATION IN VITRO OF CTL (CYTOTOXIC T LYMPHOCYTES) SPECIFIC ANTIGENIC DETERMINANTS OF HIV (HUMAN IMMUNODEFICIENCY VIRUS)

We have elicited in vitro specific CTL to HIV envelope antigens by using a recombinant (hybrid) particle (system) obtained by fusing HBenvAg (also referred to as HBsAg) with some regions of gp 120 of HIV, kindly donated by M.L. Michell of Institut Pasteur, Paris. The fusion protein, i.e. hybrid particle, is constituted of: pre-S2 region of HBenvAg and two regions of HIV type 1: region 8 of 84 aa., binding site to the CD4 molecule, and region V₃, a variable domain of 24 aa. The fusion protein is prepared using standard recombinant DNA techniques.

The HBenvAg particle is a good immunogen because it is a polymer of high molecular weight, carrying on its surface exogenous antigenic determinants. We discovered that soluble HBenvAg, differently from the most exogenous antigens, has the particular property to enter directly into the cytoplasm by membrane fusion entering class I processing pathway. In the HBenvAg/HIV system, the HBenvAg carries into the cytoplasm the glycoprotein 120 regions, allowing their presentation to CD8 T cells by MHC (Major Histocompatibility complex) class I molecules, as endogenous peptides.

The above result is demonstrated by the following. We generated specific lines by stimulation with the fusion protein (i.e. the hybrid particle), and expanded them with interleukin 2. Thereafter we tested them for their ability to proliferate in response to stimulation with the fusion proteine molecule and we cloned the resulting specific lines. The clones obtained were stained for CD4 with FITC (Fluorescein Isothiocyanate) and for CD8 with PH (Phycoerythrin) to analyze their phenotype on FACS CAN (Flow Analysis Cytometric Scanner). Some of these clones were CD8+ and they were evaluated in a chromium release essay for recognition of autologous EBV-transformed cells as target. Some CTL we obtained were specific for gp 120 of HIV. These results demonstrate that it is possible to generate in vitro CTL specific to HIV envelope, through its delivery by HBenvAg to class I processing pathway.

The HBenvAg/HIV system may be utilized for the presentation of antigenic determinants of HIV envelope to obtain a vaccine to prevent the infection and to obtain specific CTL to stop the disease.

### PROLIFERATIVE RESPONSE ESSAY

Proliferative response of lines timulated with HBsAg/HIV or gp 120 antigens. Antigen Presenting Cells (APC) were irradiated autologous Peripheral Blood Mononuclear Cells (PBMC). Peripheral Blood Lymphocytes (PBL) were isolated by Ficoll-Hipaque centrifugation from heparinized blood. The PBL were suspended in RPMI 1640 medium supplemented with 10% of FCS (Fetal Calf Serum) and were stimulated with 10µg/ml of gp 120 or 5µg/ml of HBenvAg/HIV. Five days after stimulation, ³H-tymidine incorporation of the cultures was determined by scintillation counting.

### CHROMIUM RELEASE ESSAY

Autologous target cells were labelled with 100µCi per million cells of ⁵¹Cr for 1.30 hours. After washing, labelled target cells were pulsed with HBenvAg/HIV or HBenvAg for 2.00 hours and then the target cells were incubated with effector cells for 4.00 hours at 37°C. Thereafter, 100µl of supernatant were collected and specific lysis was determined.

### FLOW CYTOMETRIC ANALYSIS

The clones were stained for CD4 with FITC (Fluorescein Isothiocyanate) and for CD8 with PH (Phycoerythrin) and they were analyzed on FACS CAN (Flow Analysis Cytometric Scanner).

## Claims

1. A process for the preparation of immunogens, characterized essentially by synthetically bonding at least one exogenous antigen to the exogenous form of hepatitis B virus envelope antigen, such immunogens being capable of eliciting, against the synthetically bonded antigen, not only the immune response of T helper (class II-restricted) lymphocytes, but also an effective response of cytotoxic (class I-restricted) T lymphocytes.

2. The process for the preparation of immunogens as per claim 1, in which the immunogens are bonded to lipoprotein membrane particles.

3. The process for the preparation of immunogens as per claim 1, in which the immunogens are incorporated into lipoprotein membrane particles.

4. The process for the preparation of vaccines as per claims 1 to 3, in which the lipoprotein membranes are liposomes.

5. The process for the preparation of vaccines as per claims 1 to 4, in which the exogenous antigens synthetically bonded to the exogenous form of hepatits B virus envelope antigen are proteins of AIDS virus.

6. The process for the preparation of immunogens as per claims 1 to 4, in which the exogenous antigens synthetically bonded to the exogenous form of hepatitis B virus envelope antigen are recombinant proteins of the AIDS virus.

7. The process for the preparation of immunogens as per claim 1 to 4, in which the exogenous antigens synthetically bonded to the exogenous form of hepatitis B virus envelope antigen are synthetic peptides of the AIDS virus.

8. The process for the preparation of immunogens as per claims 1 to 4, in which the exogenous antigens synthetically bonded to the exogenous form of hepatits B virus envelope antigen are proteins of malaria plasmodium.

9. The process for the preparation of immunogens as per claims 1 to 4, in which the exogenous antigens synthetically bonded to the exogenous form of hepatitis B virus envelope antigen are proteins of toxoplasma.

10. Immunogens, characterized by the fact of being obtainable with the process as per claims 1 to 9.
